# EUROPEAN PATENT APPLICATION

(11) **EP 4 212 548 A1**
(43) Date of publication of application: **19.07.2023**
(21) Application number: 21867109.7
(22) Date of filing: 08.09.2021
(51) Int. Cl.: C07K 16/10, A61P 31/16, A23L 33/18, A61K 39/00

(54) **ANTIVIRAL COMPOSITION AGAINST INFLUENZA A VIRUS**

(30) Priority: 08.09.2020 US 202063075456 P
(71) Applicant: Novelgen Co., Ltd., Suwon-si, Gyeonggi-do 16230 (KR)
(72) Inventor: KIM, Tai Hyun, Suwon-si, Gyeonggi-do 16420 (KR); KIM, Young Jun, Andong-si, Gyeongsangbuk-do 36719 (KR); OH, Kwang Ji, Daejeon 34010 (KR); LEE, Gun Sup, Jeonju-si, Jeollabuk-do 54972 (KR); LEE, Yong Jun, Gwacheon-si, Gyeonggi-do 13804 (KR)
(74) Representative: dompatent von Kreisler Selting Werner - Partnerschaft von Patent- und Rechtsanwälten mbB
(86) International application number: PCT/KR2021/012191
(87) International publication number: WO 2022/055243

(57) **Abstract**

The present invention provides an antiviral composition for influenza A virus, and more particularly, an antiviral composition, including an antibody or fragment thereof, which includes: a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6, so as to exhibit excellent antiviral effects to influenza A virus, while achieving effects of preventing, treating or improving diseases derived from influenza A virus.

## Description

### [Technical Field]

The present invention relates to an antiviral composition for influenza A virus.

### [Background Art]

Influenza, commonly known as 'the flu', is a contagious febrile respiratory disease caused by an influenza virus. Influenza A and B viruses spread to people and cause seasonal flu outbreaks each year. According to the World Health Organization (WHO), influenza virus infection causes about 3 to 5 million severe cases and about 290,000 to 650,000 deaths. The WHO recommends annual influenza vaccination for high-risk groups, and vaccines are generally effective to three to four types of influenza. However, due to rapid evolution of viruses, a vaccine made in one year may not be useful the following year. The genomes of influenza A and B viruses can be mutated in two ways: antigenic drift and antigenic shift, therefore, the existing vaccines or antiviral agents show low antiviral efficacy to viruses. Accordingly, even if a mutation occurs in the genome of the influenza virus, there is a need to develop a material exhibiting excellent antiviral efficacy.

### [Summary of Invention]

### [Problems to be Solved by Invention]

An object of the present invention is to provide an antiviral composition for influenza A virus.

In addition, another object of the present invention is to provide a pharmaceutical composition for prevention or treatment of influenza A virus-infectious diseases.

Further, another object of the present invention is to provide a food composition for prevention or treatment of influenza A virus-infectious diseases.

### [Means for Solving Problems]

1. An antiviral composition for influenza A virus, including an antibody or fragment thereof, which includes: a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6.
2. The antiviral composition according to the above 1, wherein the antibody or fragment thereof includes a heavy chain variable region including an amino acid sequence of SEQ ID NO: 7.
3. The antiviral composition according to the above 1, wherein the antibody or fragment thereof includes a light chain variable region including an amino acid sequence of SEQ ID NO: 8.
4. The antiviral composition according to the above 1, wherein the fragment is scFv including an amino acid sequence of SEQ ID NO: 9.
5. The antiviral composition according to the above 1, wherein the influenza A virus is at least one selected from the group consisting of influenza A virus H1 subtype, influenza A virus H2 subtype, influenza A virus H5 subtype and influenza A virus H6 subtype, influenza A virus H3 subtype, influenza A virus H4 subtype, influenza A virus H14 subtype, and variants thereof.
6. The antiviral composition according to the above 1, wherein the influenza A virus is at least one selected from the group consisting of influenza A virus N1 subtype, influenza A virus N2 subtype, and variants thereof.
7. The antiviral composition according to the above 1, wherein the influenza A virus is at least one of H1N1 subtype, H3N2 subtype, and variants thereof.
8. The antiviral composition according to the above 1, wherein the influenza A virus is an oseltamivir-resistant influenza A virus.
9. A pharmaceutical composition for prevention or treatment of influenza A virus-infectious diseases, including the composition of any one of the above 1 to 8.
10. The pharmaceutical composition according to the above 9, which is used for treating influenza A virus-infectious disease.
11. A food composition for prevention or treatment of influenza A virus-infectious diseases, including the composition of any one of the above 1 to 8.
12. The food composition according to the above 11, wherein the composition is used for improving influenza A virus-infectious diseases.

### [Advantageous effects]

The antibody or fragment thereof included in the antiviral composition of the present invention may penetrate into virus-infected cells, bind to influenza A virus RNA, and degrade the same.

The antiviral composition of the present invention may exhibit excellent antiviral effects on influenza A virus by specifically binding to influenza A virus and hydrolyzing the same, while achieving effects of preventing, treating or improving influenza A virus-infectious diseases.

The antiviral composition of the present invention may exhibit excellent antiviral efficacy to various subtypes of influenza A virus or variants thereof.

The antiviral composition of the present invention may exhibit excellent antiviral efficacy to oseltamivir-resistant influenza A virus.

### [Brief Description of Drawings]

FIG. 1 illustrates a sequence of an antibody or fragment thereof according to an embodiment of the present invention.
FIG. 2 illustrates a partial structure of a recombinant vector for production of the antibody fragment according to an embodiment of the present invention.
FIG. 3 illustrates an intracellular penetration activity of the antibody fragment according to an embodiment.
FIG. 4 illustrates a viral RNA (NA and NP) hydrolytic activity of the antibody fragment according to an embodiment of the present invention.
FIG. 5 illustrates results of confirming intracellular toxicity of the antibody fragment according to an embodiment of the present invention through MTT analysis.
FIG. 6 illustrates a schematic diagram and test results of an antiviral effect (cell viability) of 3D8 scFv pre-treatment for influenza A virus strains (H1N1/PR8, H3N2/X-31 and H1N1/H275Y) (*P < 0.05 , **P < 0.01, ***P < 0.001) .
FIG. 7 illustrates a schematic diagram and test results of the antiviral effect (cell viability) of 3D8 scFv post-treatment for influenza A virus strains (H1N1/PR8, H3N2/X-31 and H1N1/H275Y) (*P < 0.05, **P < 0.01, ***P < 0.001) .
FIG. 8 illustrates test results of the antiviral effect (viral RNA/protein expression level) of the 3D8 scFv pre/post-treatment for the H1N1/PR8 strain.
FIG. 9 illustrates test results of the antiviral effect (viral RNA/protein expression level) of the 3D8 scFv pre/post-treatment for the H3N2/X-31 strain.
FIG. 10 illustrates results of the antiviral effect (viral RNA/protein expression level) of 3D8 scFv pre/post-treatment for the H1N1/H275Y strain.
FIG. 11 illustrates results of detecting viral protein HA in cells infected with influenza virus after 3D8 scFv was pre-treated (nucleus: DAPI (blue), 3D8 scFv: Alexa flour 488 (green), virus HA: TRITC (red)).
FIG. 12 illustrates results of detecting viral protein HA in cells infected with influenza virus after 3D8 scFv was post-treated (nucleus: DAPI (blue), 3D8 scFv: Alexa flour 488 (green), virus HA: TRITC (red)).
FIG. 13 illustrates a schematic diagram of the 3D8 scFv treatment and H1N1/H275Y infection protocol in mice for *in vivo* antiviral activity test.
FIG. 14 illustrates results of assessing *in vivo* pharmacological properties of the antibody fragment according to an embodiment of the present invention.
FIG. 15 illustrates results of assessing *in vivo* pharmacological properties of the antibody fragment according to another embodiment of the present invention.
FIG. 16 illustrates the survival rate (cell viability) of influenza virus-infected mice.
FIG. 17 illustrates an average body weight of influenza virus-infected mice.
FIG. 18 illustrates morphological comparison of lungs and lung lesion scores of influenza virus-infected mice.
FIG. 19 illustrates histopathological comparison of lungs and microscopic lung lesion scores of influenza virus-infected mouse lung sections.
FIG. 20 illustrates results of qRT-PCR analysis of viral RNA (HA, NP) and IFN-β expression in influenza virus-infected mice.
FIG. 21 illustrates viral titers of influenza virus-infected mice through plaque assay.
FIG. 22 illustrates results of confirming a degree of decrease in viral protein HA in the lungs of influenza virus-infected mice (*P < 0.05, **P < 0.01, ***P < 0.001).

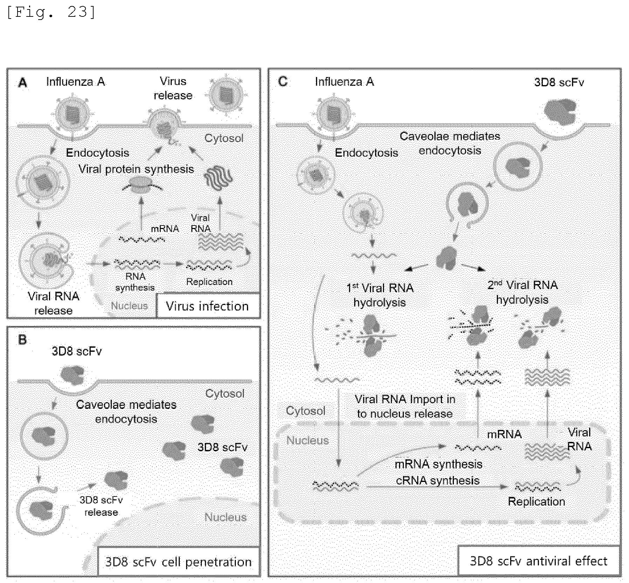

FIG. 23 illustrates a schematic diagram of a proposed model of the functional mechanism of an antibody fragment according to an embodiment of the present invention, wherein: FIG. 23(A) shows a replication pathway of the influenza virus; FIG. 23(B) shows a cell penetration mechanism of the antibody fragment according to an embodiment of the present invention; and FIG. 23(C) shows a hydrolysis mechanism of influenza A virus RNA in the cytoplasm by the antibody fragment according to an embodiment of the present invention.
FIG. 24 illustrates 16 influenza A virus subtypes defined by hemagglutinin (HA).

### [Mode for Carrying out Invention]

Hereinafter, the present invention will be described in detail.

The present invention provides an antiviral composition for influenza A virus, including an antibody or fragment thereof which includes: a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6.

The term "complementarity determining region (CDR)" refers to a region involved in antigen recognition, wherein the specificity of the antibody to an antigen is determined when a sequence of this region changes. As used herein, the VH CDR refers to a heavy chain complementarity determining region present in a heavy chain variable region, and the VL CDR refers to a light chain complementarity determining region present in a light chain variable region.

The term "variable region" refers to a region that shows sequence variation while performing a function of specifically binding to an antigen, and CDR1, CDR2 and CDR3 exist in the variable region. A framework region (FR) portion exists between the CDRs, which may serve to support the CDRs. Irrespective of the framework regions, CDRs may be important regions that allow an antibody or fragment thereof to exhibit specific functions.

The antibody or fragment thereof may include the heavy chain variable region which includes: a heavy chain CDR1 (VH CDR1) represented by SEQ ID NO: 1, VH CDR2 represented by SEQ ID NO: 2, and VH CDR3 represented by SEQ ID NO: 3; and a light chain variable region which includes: a light chain CDR1 (VL CDR1) represented by SEQ ID NO: 4, VL CDR2 represented by SEQ ID NO: 5, and VL CDR3 represented by SEQ ID NO: 6.

The antibody or fragment thereof may include a heavy chain variable region which includes an amino acid sequence of SEQ ID NO: 7. According to one embodiment, the antibody or fragment thereof may include a heavy chain variable region consisting of the amino acid sequence of SEQ ID NO: 7.

The antibody or fragment thereof may include a light chain variable region which includes an amino acid sequence of SEQ ID NO: 8. According to one embodiment, the antibody or fragment thereof may include a light chain variable region consisting of the amino acid sequence of SEQ ID NO: 8.

A fragment of antibody (`antibody fragment') may be at least one selected from the group consisting of single chain antibodies, Fv, scFv, di-scFv, Fab, Fab', F(ab')₂, Fd, LC, dAb, CDR, scFv-Fc, and diabodies.

The antibody fragment may include an amino acid sequence of SEQ ID NO: 9. According to one embodiment, the antibody fragment may consist of the amino acid sequence of SEQ ID NO: 9. According to one embodiment, the antibody fragment may consist of scFv including the amino acid sequence of SEQ ID NO: 9.

An antibody or fragment thereof may be a humanized antibody or fragment thereof; a chimeric antibody or fragment thereof. Humanized antibodies may be prepared by methods known in the art.

According to an embodiment, the antibody fragment may be a humanized scFv including CDR sequences of the scFv of SEQ ID NO: 9.

The antibody or fragment thereof may penetrate into the cytoplasm of a virus-infected host cell. The penetrating effect of the antibody or fragment thereof into the cytoplasm may be considered to be due to Caveolae mediated endocytosis.

The antibody or fragment thereof according to the present invention may specifically bind to a nucleic acid of influenza A virus. In particular, the antibody or fragment thereof according to the present invention may specifically bind to RNA of influenza A virus. The antibody or fragment thereof according to the present invention has nuclease activity and can degrade RNA and DNA of viruses.

The antibody or fragment thereof according to the present invention can penetrate into the cytoplasm of a host infected with influenza A virus, and it may induce a decrease in amount of viral gene replication and a decrease in the viral protein by removing naked viral RNA from the infected cells, while having the efficacy of virus treatment.

Influenza A virus as a surface antigen may be divided into hemagglutinin (HA) subtype and neuraminidase (NA) subtype or variants thereof.

The mutant may be a mutant of influenza A virus subtype, for example, may be one in which a mutation in hemagglutinin and/or neuraminidase of the influenza A virus occurs.

Specifically, the mutant is a mutant of the influenza A virus subtype, and may be one in which a mutation in neuraminidase of the influenza A virus occurs thus to exhibit Oseltamivir (Tamiflu) resistance. For example, the variant may be H1N1/H275Y. H1N1/H275Y is a protein mutation resulting from H1N1/pdm09.

The functional mechanism of Tamiflu is to inhibit the spike protein of neuraminidase of H1N1 and thus prevent spreading thereof. H1N1/H275Y in which amino acid mutation of H275Y of neuraminidase of H1N1 occurs has very weak binding force between neuraminidase and Tamiflu, thereby exhibiting Tamiflu resistance. Hemagglutinin may serve to recognize the surface of a host cell and penetrate into the cell. Further, it may induce different inflammatory responses inside the cell and serve to self-replication.

Influenza A virus may be formed in various subtypes defined by hemagglutinin or variants thereof.

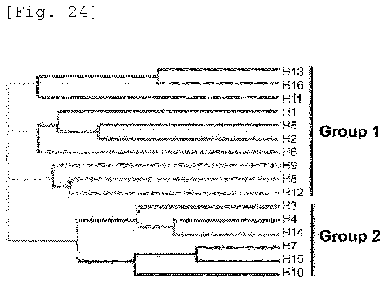

The subtypes defined by hemagglutinin may include H1 subtype, H2 subtype, H3 subtype, H4 subtype, H5 subtype, H6 subtype, H7 subtype, H8 subtype, H9 subtype, H10 subtype, H11 subtype, H12 subtype, H13 subtype, H14 subtype, H15 subtype, and H16 subtype (FIG. 24). Influenza A virus H1, H2 and H3 subtypes generally cause diseases in humans.

Influenza A virus may be any of subtypes, for example: a subtype including H1 hemagglutinin, a subtype including H2 hemagglutinin, a subtype including H3 hemagglutinin, a subtype including H4 hemagglutinin, a subtype including H5 hemagglutinin, a subtype including H6 hemagglutinin, a subtype including H7 hemagglutinin, a subtype including H8 hemagglutinin, a subtype including H9 hemagglutinin, a subtype including H10 hemagglutinin, a subtype including H11 hemagglutinin, a subtype including H12 hemagglutinin, a subtype including H13 hemagglutinin, a subtype including H14 hemagglutinin, a subtype including H15 hemagglutinin, a subtype including H16 hemagglutinin, and/or variants thereof.

The influenza A virus may be at least one of the remaining 15 subtypes except for H1 subtype among 16 subtypes defined by hemagglutinin (HA).

For example, the antibody or fragment thereof according to the present invention may exhibit antiviral efficacy to at least one selected from the group consisting of influenza A virus H1 subtype, influenza A virus H2 subtype, influenza A virus H5 subtype, influenza A virus H6 subtype, influenza A virus H3 subtype, influenza A virus H4 subtype, influenza A virus H14 subtype, and variants thereof. According to one embodiment, the antibody or fragment thereof according to the present invention may exhibit antiviral efficacy to at least one selected from the group consisting of influenza A virus H1 subtype, influenza A virus H3 subtype, and variants thereof.

Further, the influenza A virus may include a variety of subtypes and variants thereof defined by neuraminidase (NA) .

Neuraminidase is an enzyme acting as a 'scissor' so that the virus proliferated in the host cell can break through the cell membrane, and may play a key role in the spread of the virus.

For example, the influenza A virus may include influenza A virus N1 subtype, N2 subtype, N3 subtype, N4 subtype, N5 subtype, N6 subtype, N7 subtype, N8 subtype, N9 subtype, and variants thereof.

The influenza A virus may be a subtype including N1 neuraminidase, a subtype including N2 neuraminidase, and variants thereof.

According to one embodiment, the antibody or fragment thereof according to the present invention may exhibit antiviral efficacy to at least one selected from the group consisting of influenza A virus N1 subtype, influenza A virus N2 subtype, and variants thereof.

According to one embodiment, the antibody or fragment thereof according to the present invention may exhibit antiviral efficacy to at least one selected from the group consisting of H1N1 subtype, H3N2 subtype, and variants thereof. For example, the antibody or fragment thereof according to the present invention may exhibit antiviral efficacy to H1N1/PR8, H3N2/X-31, H1N1/H275Y virus strains.

Further, the present invention provides a pharmaceutical composition for prevention or treatment of influenza A virus-infectious diseases, which includes the above-described antiviral composition.

Since the antiviral composition, the antibody or fragment thereof, and influenza A virus have been described above, and therefore will not be described in detail.

Specifically, the present invention provides a pharmaceutical composition capable of treating influenza A virus-infectious diseases, which includes the above-described antiviral composition.

The term "prevention" refers to any action that suppresses a disease caused by a virus or delays the onset of the disease.

The term "treatment" refers to any action that improves or beneficially changes symptoms of a disease caused by a virus.

Influenza A virus may be a subtype defined by hemagglutinin (HA) or a variant thereof. The subtypes defined by hemagglutinin (HA) may include H1 subtype, H2 subtype, H3 subtype, H4 subtype, H5 subtype, H6 subtype, H7 subtype, H8 subtype, H9 subtype type, H10 subtype, H11 subtype, H12 subtype, H13 subtype, H14 subtype, H15 subtype and H16 subtype (FIG. 24). Further, the influenza A virus may include a variety of subtypes and variants thereof defined by neuraminidase (NA). For example, the influenza A virus may include influenza A virus N1 subtype, N2 subtype, N3 subtype, N4 subtype, N5 subtype, N6 subtype, N7 subtype, N8 subtype, N9 subtype, and variants thereof.

According to one embodiment, the influenza A virus may be at least one selected from the group consisting of H1N1 subtype, H3N2 subtype, and variants thereof.

Influenza A virus-infectious diseases may include fever, vomiting, diarrhea, malaise, cough, dyspnea, pneumonia, headache, muscle pain, sputum, sore throat, flu, cold, pain/pressure in the chest, and sudden dizziness, however, they are not limited thereto so long as they are diseases caused by viral infection.

The influenza A virus-infectious disease is not limited so long as it is a disease with clinical symptoms of influenza A.

The pharmaceutical composition of the present invention may be used alone or in combination with conventional methods using surgery, drug therapy, and biological response modifiers for prevention and treatment of diseases.

The pharmaceutical composition may include an additional component effective to influenza A virus infection, and the additional component may be any component known to be effective to viral infection, which includes compounds, natural products, proteins, peptides, nucleic acids and the like.

The pharmaceutical composition may further include suitable carriers, excipients and diluents commonly used in the preparation of the composition. Such carriers, excipients and diluents may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil or the like.

The pharmaceutical composition of the present invention may be formulated in oral dosage forms such as powders, granules, tablets, capsules, suspensions, emulsions, syrups, aerosols, external preparations, suppositories, and sterile injection solutions according to conventional methods, and such formulations may be performed by a method commonly performed in the pharmaceutical field. In the case of formulation, it may be prepared by additionally applying diluents or excipients such as commonly used fillers, extenders, binders, wetting agents, disintegrants, and surfactants.

Solid preparations for oral administration may include tablets, pills, powders, granules, capsules, etc., and such solid preparations may include at least one excipient, for example, starch, calcium carbonate, sucrose or lactose, gelatin, etc. In addition to simple excipients, lubricants such as magnesium stearate and talc are also used. Liquid formulations for oral administration may include suspensions, internal solutions, emulsions, syrups, etc., and various excipients such as wetting agents, sweeteners, fragrances, and preservatives, other than water and liquid paraffin, which are commonly used simple diluents, may be included. Formulations for parenteral administration may include sterile aqueous solutions, non-aqueous solutions, suspensions, emulsions, freeze-dried preparations and suppositories. As the non-aqueous agent and suspending agent, propylene glycol, polyethylene glycol, vegetable oils such as olive oil, and injectable esters such as ethyl oleate may be used. As the base of the suppository, witepsol, macrogol, tween, cacao butter, laurin, glycerogelatin, etc. may be used.

Further, the present invention provides a food composition for prevention or improvement of influenza A virus-infectious diseases, which includes the above-described antiviral composition.

The antiviral compositions, antibodies or fragments thereof, influenza A virus and diseases have been described above, and therefore will not be described in detail.

Specifically, the present invention provides a food composition capable of improving influenza A virus-infectious diseases, which includes the above-described antiviral composition.

The term "improvement" refers to any action that reduces the symptoms of a disease caused by a virus.

The food composition may be used together with other foods or food ingredients, and may be appropriately used according to a conventional method.

A mixing amount of the active ingredient included in the food composition may be suitably determined according to the purpose of its use (for prevention or improvement).

The type of food composition is not particularly limited and may include, for example, meat, sausage, bread, chocolate, candy, snacks, confectionery, pizza, ramen, other noodles, gums, dairy products including ice cream, various soups, beverages, tea, drinks, alcoholic beverages and vitamin complexes.

Like conventional foods, the food composition may further include additional ingredients such as flavoring agents or natural carbohydrates other than active ingredients. The natural carbohydrates may include: monosaccharides such as glucose and fructose; disaccharides such as maltose and sucrose; polysaccharides such as dextrin and cyclodextrin; as well as a sugar alcohol such as xylitol, sorbitol, or erythritol. Examples of the sweetener may include natural sweeteners such as taumatin and stevia extract; and synthetic sweeteners such as saccharin and aspartame.

In addition to the above, the health food of the present invention may include various nutrients, vitamins, electrolytes, flavoring agents, colorants, pectic acid and its salts, alginic acid and its salts, organic acids, protective colloidal thickeners, pH adjusters, stabilizers, preservatives, glycerin, alcohol, carbonation agents used in carbonated beverages and the like. In addition, it may contain the pulp for production of natural fruit juice, fruit juice beverage and vegetable beverage.

Further, the present invention provides a method for preventing or treating an influenza A virus-infectious disease, which includes administering the above-described antiviral composition to a subject. In particular, the present invention provides a method for treating an influenza A virus-infectious disease, which includes administering the above-described antiviral composition to a subject.

The subject may be a human and/or a non-human animal, and specifically, a mammal capable of exhibiting beneficial effects by treatment using the drug of the present invention.

The subject may include all subjects who have or are at risk of having symptoms of influenza A virus-infectious diseases (e.g., fever, malaise, cough, dyspnea, pneumonia, sputum, sore throat, flu, cold, etc.). The influenza A virus is the same as described above, and therefore will not be described in detail.

An amount effective for preventing or treating influenza A virus-infectious diseases means an amount capable of achieving a desired outcome in a subject in need thereof, or an amount capable of providing objective or subjective benefits to a subject in need thereof.

The amount effective for preventing or treating influenza A virus-infectious diseases may be single or multiple doses. An effective amount for preventing or treating an influenza A virus-infectious disease may be an amount when the drug of the present invention is provided alone, but may also be an amount when provided in combination with one or more other compositions (e.g., other respiratory disease treatment agents, etc.).

Numerical values described herein should be construed to include equivalent ranges unless otherwise specified.

Further, the present invention provides the use of a pharmaceutical composition which includes the above-described antiviral composition for prevention or treatment of an influenza A virus-infectious disease.

Specifically, the present invention provides the use of a pharmaceutical composition which includes the above-described antiviral composition for treating an influenza A virus-infectious disease.

Hereinafter, examples will be given to describe the present invention in detail.

### 1. Experimental materials: Cell lines and viruses

MDCK cells were preserved in Eagle's minimal essential medium (EMEM) containing 5% fetal bovine serum (Gibco, Cergy Pontois, France), 100 U/ml penicillin and 100 ug/ml streptomycin (Hyclone, Logan, UT, USA). HeLa and Vero cells were cultured in DMEM (Dulbecco's Modified Eagle's medium), and A549 cells were cultured in Roswell park Memorial Institute 1640 medium (RPMI 1640 medium; Hyclone) containing the same serum as the MDCK culture medium. The cell line was purchased from the Korea Cell Line Bank and maintained at 37 °C and 5% CO₂. Influenza strains A/Puerto Rico/8/34 (H1N1/PR8) and A/X-31 (H3N2/X-31) were provided by Professor Kweon (Sungkyunkwan University, Korea). Further, Pandemic H1N1/H275Y NA mutant virus (A/Korea/2785/2009pdm: NCCP 42017; Oseltamivir-resistant) was obtained from the National Pathogen Resources Bank. H1N1/H275Y is a variant resulting from H1N1/pdm09. Viruses were proliferated in the allantoic sacs of 9-day-old eggs at 37 °C for 3 days. Allantoic fluid was harvested and removed using density gradient centrifugation. Viral titers were determined using plaque assay.

### 2. Expression and purification of antibody fragment

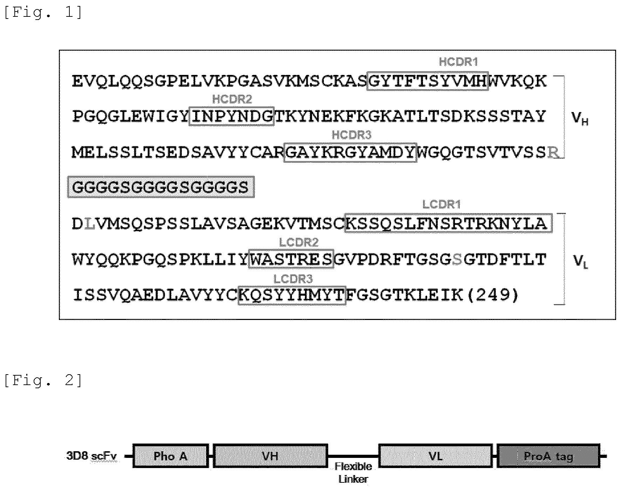

Using *E. coli* BL21 cell as a host, scFv having the amino acid sequence of SEQ ID NO: 9 of Table 1 below was produced (see FIG. 1). ScFv-related sequences (scFv sequences; VH, VL, CDR sequences thereof; and gene sequences thereof) are described in Table 1 below. Specifically, using the pIg20-3D8 plasmid-transformed *Escherichia coli* (BL21[DE3]pLysE) strain in Luria-Bertani (LB) broth containing 100 µg/ml ampicillin and 20 ug/ml chloramphenicol at 37 °C, the antibody fragment (3D8 scFv) was expressed (see FIG. 2), and 1 mM isopropyl 1-thiol-β-D-galactopyranoside (IPTG) was added for induction at 26 °C for 18 hours. The cell culture supernatant was obtained by centrifugation at 5,200 x g at 4 °C for 20 minutes, and then passed through a 0.22 um filter. 3D8 scFv was purified from the supernatant using an IgG Sepharose 6 fast flow (GE Healthcare, Malborough, MA, USA) affinity column. The column was washed with 10 bed volumes of PBS (pH 7.4) and then with 10 bed volumes of 5 mM ammonium acetate (pH 5.0). Next, the 3D8 scFv was eluted with 0.1 M acetic acid (pH 3.4). The eluted fractions were neutralized with 0.1 volumes of 1 M Tris-HCl (pH 9.0). The concentration of the purified 3D8 scFv was determined based on the extinction coefficient at 280 nm. Proteins were analyzed by electrophoresis on a 12% SDS polyacrylamide gel and detected by Coomassie blue staining.

**[TABLE 1]**

| **Division** | **Amino acid sequence** | **SEQ ID** N**o.** |
|---|---|---|
| VH CDR1 | GYTFTSYVMH | SEQ ID NO: 1 |
| VH CDR2 | INPYNDG | SEQ ID NO: 2 |
| VH CDR3 | GAYKRGYAMDY | SEQ ID NO: 3 |
| VL CDR1 | KSSQSLFNSRTRKNYLA | SEQ ID NO: 4 |
| VL CDR2 | WASTRES | SEQ ID NO: 5 |
| VL CDR3 | KQSYYHMYT | SEQ ID NO: 6 |
| VH | EVQLQQSGPELVKPGASVKMSCKASGYTFTSYVMHW VKQKPGQGLEWIGYINPYNDGTKYNEKFKGKATLTS DKSSSTAYMELSSLTSEDSAVYYCARGAYKRGYAMD YWGQGTSVTVSSR | SEQ ID NO: 7 |
| VL | DLVMSQSPSSLAVSAGEKVTMSCKSSQSLFNSRTRK NYLAWYQQKPGQSPKLLIYWASTRESGVPDRFTGSG SGTDFTLTISSVQAEDLAVYYCKQSYYHMYTFGSGT KLEIK | SEQ ID NO: 8 |
| 3D8 scFv | EVQLQQSGPELVKPGASVKMSCKASGYTFTSYVMHW VKQKPGQGLEWIGYINPYNDGTKYNEKFKGKATLTS DKSSSTAYMELSSLTSEDSAVYYCARGAYKRGYAMD YWGQGTSVTVSSRGGGGSGGGGSGGGGSDLVMSQSP SSLAVSAGEKVTMSCKSSQSLFNSRTRKNYLAWYQQ KPGQSPKLLIYWASTRESGVPDRFTGSGSGTDFTLT ISSVQAEDLAVYYCKQSYYHMYTFGSGTKLEIK | SEQ ID NO: 9 |
| 3D8 scFv gene sequence | GAGGTCCAGCTGCAGCAGTCTGGACCTGAGCTGGTA AAGCCTGGGGCTTCAGTGAAGATGTCCTGCAAGGCT TCTGGATACACATTCACTAGCTATGTTATGCACTGG GTGAAGCAGAAGCCTGGGCAGGGCCTTGAGTGGATT GGATATATTAATCCTTACAATGATGGTACTAAGTAC AATGAGAAGTTCAAAGGCAAGGCCACACTGACTTCA GACAAATCCTCCAGCACAGCCTACATGGAGCTCAGC AGCCTGACCTCTGAGGACTCTGCGGTCTATTACTGT GCAAGAGGGGCCTATAAAAGGGGATATGCTATGGAC TACTGGGGTCAAGGAACCTCAGTCACCGTCTCCTCT AGAGGTGGGGGCGGTTCGGGTGGCGGGGGCTCGGGC GGGGGTGGCTCAGATCTTGTGATGTCACAGTCTCCA TCCTCCCTGGCTGTGTCAGCAGGAGAGAAGGTCACT ATGAGCTGCAAATCCAGTCAGAGTCTGTTCAACAGT AGAACCCGAAAGAACTACTTGGCTTGGTACCAGCAG AAACCAGGGCAGTCTCCTAAACTGCTGATCTACTGG GCATCCACTAGGGAATCTGGGGTCCCTGATCGCTTC ACAGGCAGTGGATCTGGGACAGATTTCACTCTCACC ATCAGCAGTGTGCAGGCTGAAGACCTGGCAGTTTAT TACTGCAAGCAATCTTATTATCACATGTATACGTTC GGATCGGGGACCAAGCTGGAAATAAAA | SEQ ID NO: 10 |

### 3. Analysis of cell penetration activity of antibody fragment

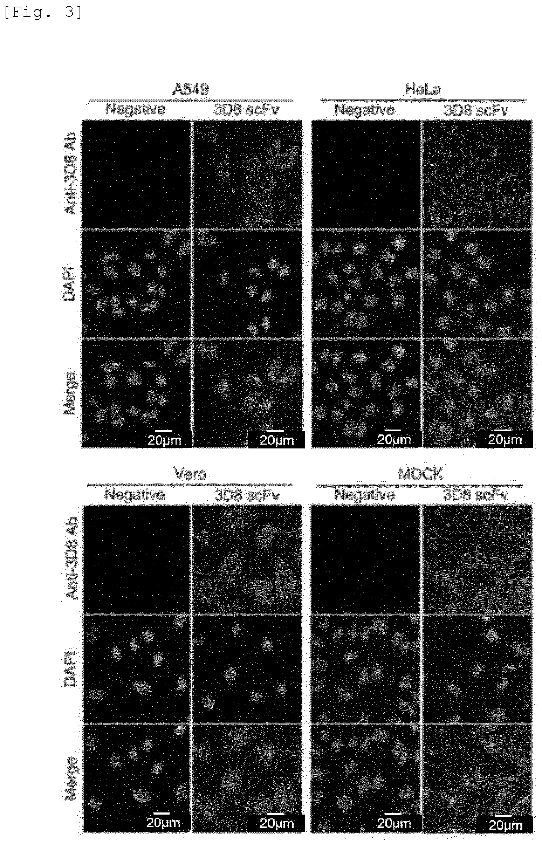

First, cell penetration activity as an important prerequisite for antiviral effects of 3D8 scFv was investigated. A549, HeLa, Vero and MDCK cells (2 × 10⁴) were cultured in 8-well chamber slides (Nunc Lab-Tek). Cells were treated with 3 µM of 3D8 scFv and incubated at 37 °C under 5% CO₂ for 24 hours. Next, the slides were washed twice with PBS and fixed in methanol cold at 25 °C for 15 minutes. Samples were permeabilized for 5 minutes using Intracellular Staining Perm wash buffer (BioLegend, San Diego, CA, USA). After blocking with 1% BSA (in PBST) for 1 hour, the samples were incubated with monoclonal mouse anti-3D8 scFv antibody (1:1000 dilution; AbClon, Seoul, Korea) at 4 °C for 24 hours. Then, the samples were incubated with goat anti-mouse IgG Alexa Fluor 488 secondary antibody (1:1000 dilution; Abcam, Cambridge, MA, USA) at 25 °C for 1 hour, followed by mounting the slides on antifade mounting medium containing DAPI (Vectashield, Burlingame, CA, USA). The samples were then visualized using a Zeiss LSM 700 confocal microscope. Nuclei were detected with DAPI staining (blue) and 3D8 scFv was visualized with an antibody conjugated with Alexa flour 488 fluorescent dye (green). 3D8 scFv was detected in the cytoplasm of all cell lines within 24 hours after treatment (FIG. 3).

### 4. Viral RNA degradation activity of antibody fragment

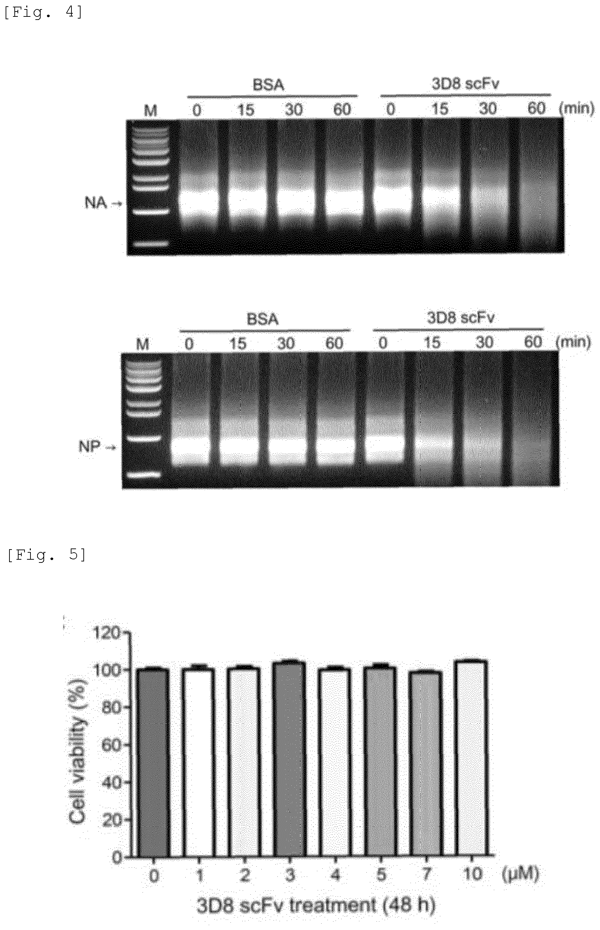

Total RNA was isolated from H1N1/PR8 and viral cDNA was synthesized. Sequences corresponding to NA and NP were amplified using PCR (Table 2) and then cloned into pGEM-T Easy (Promega, Madison, WI, USA) which is a vector carrying T7 and SP6 promoters. Neuramindase (NA) and Nucleoprotein (NP) RNAs were synthesized using a HiScribe T7 *in vitro* transcription kit (New England BioLabs, Ipswich, MA, USA) and then incubated along with 3D8 scFv purified protein (0.5 µg) in TBS containing 0.1 mM MgCl₂ at 37 °C for 1 hour. After terminating the reaction by adding 10X loading buffer (Takara, Otsu, Shinga, Japan), the reaction was analyzed by electrophoresis on a 1% agarose gel followed by staining the product with ethidium bromide. As a result of the analysis, it was confirmed that viral RNAs were degraded by 3D8 scFv treatment (FIG. 4).

**[TABLE 2]**

| **Gene** | **Forward (5'->3')** | **Reverse (5'->3')** | **Accessio n No.** |
|---|---|---|---|
| Hemagglut inin (HA) | GCTACCATGCGAACAATTC AAC (SEQ ID NO: 11) | CTGCACTGCAAAGATCCATT AG (SEQ ID NO: 12) | NC_00201 7.1 |
| Neuramind ase (NA) | CCATTGGATCAATCTGTCT GG (SEQ ID NO: 13) | TGTCAATGGTGAATGGCAAC TC (SEQ ID NO: 14) | NC_00201 8.1 |
| Nucleopro tein (NP) | ACTGATGGAGAACGCCAGA AT (SEQ ID NO: 15) | ATGTCAAAGGAAGGCACGAT C (SEQ ID NO: 16) | NC_00201 9.1 |

### 5. Cytotoxicity analysis of antibody fragments

MDCK cells (2 × 10⁴) were cultured in 96-well plates. Cells were treated with 3D8 scFv (1 to 10 µM) and incubated at 37 °C under 5% CO₂ for 48 hours. Then, 10 µl of MTT solution was added and the cells were incubated at 37 °C under 5% CO₂ for 4 hours. Formazan crystals formed were dissolved with DMSO and the absorbance of each well was read at 595 nm using an ELISA microplate reader (TECAN, Mannedorf, Switzerland).

Cytotoxicity was determined by treating MDCK cells with different concentrations of 3D8 scFv (0 to 10 µM). A minimal cytotoxicity of less than 5% was observed at all concentrations, indicating that the toxicity of the 3D8 scFv was maintained to a minimum at the concentration at which the experiment was performed (FIG. 5).

### 6. Efficacy of antibody fragment to increase viability of influenza A virus-infected cell

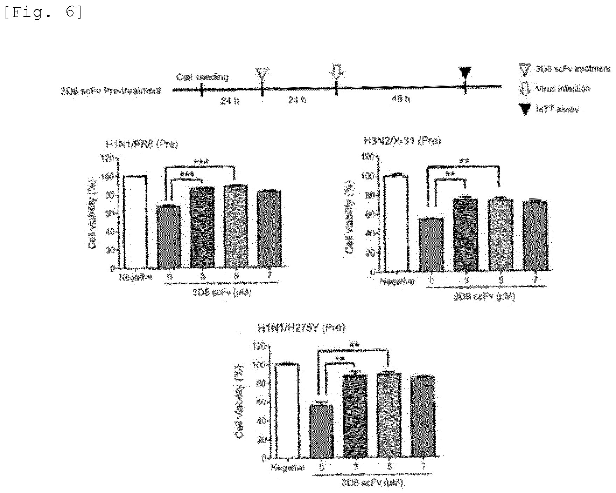

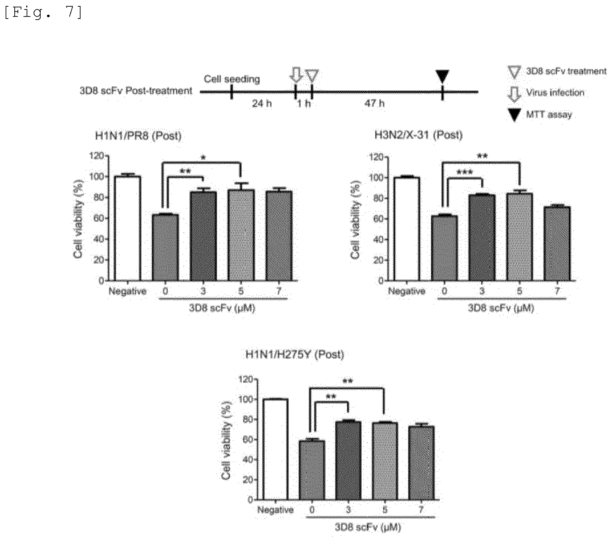

MDCK cells (2 × 10⁴) were cultured in 96-well plates and treated as follows: (i) 3D8 scFv pre-treatment: 3D8 scFv (3, 5, 7 µM) was added to MDCK cells at 37 °C under 5% CO₂. After 24 hours of treatment, 3D8 scFv-treated cells were independently infected using three influenza virus strains (H1N1/PR8, H3N2/X-31 and H1N1/H275Y) (MOI = 0.1) in MEM-free medium at 37°C for 1 hour. The infection medium was then removed and the cells were cultured in serum-free MEM-free medium (1% BSA) containing tosylphenylalanyl chloromethyl ketone (TPCK)-treated trypsin (1 µg/ml) at 37 °C under 5% CO₂ for 48 hours (the upper part of FIG. 6); (ii) 3D8 scFv post-treatment: MDCK cells were independently infected using three influenza virus strains (H1N1/PR8, H3N2/X-31 and H1N1/H275Y) (MOI = 0.1) in MEM-free medium at 37 °C for 1 hour. After infection, the infection medium was removed. After replacing the medium with MEM-free medium (1% BSA) containing 3D8 scFv (3, 5, 7 µM) and TPCK-treated trypsin (1 pg/ml), culturing was performed at 37 °C under 5% CO₂ for 47 hours (the upper part of FIG. 7). Then, 10 µl of MTT solution was added to each well and the cells were incubated at 37° C under 5% CO₂ for 4 hours. The formed formazan crystals were dissolved with DMSO and the absorbance at 595 nm was measured using an ELISA microplate reader. For reference, when cells infected with each of the three types of virus (MOI = 0.1) were treated with different concentrations of oseltamivir, H1N1/H275Y-infected cells exhibited a drug-resistant phenotype, whereas H1N1/PR8 and H3N2/X-31-infected cells did not exhibit the drug-resistant phenotype.
(i) When 3D8 scFv was pre-treated, the survival rate of the 3D8 scFv untreated group (0 µM) in H1N1/PR8-infected cells was 66.8%, while the survival rate of the group pre-treated with 5 µM of 3D8 scFv was about 88.3% (H1N1/PR8(Pre) in FIG. 6). Further, the survival rate of the 3D8 scFv untreated group (0 µM) in H3N2/X-31-infected cells was about 54.5%, while the survival rate of the group pre-treated with 3D8 scFv at 3 µM was about 74.4% (H3N2/X-31(Pre) in FIG. 6). In H1N1/H275Y-infected cells, the survival rate of the 3D8 scFv untreated group (0 µM) was about 55.6%, while the survival rate of all 3D8 scFv-treated groups was about 85% (H1N1/H275Y(Pre) in FIG. 6). (ii) When 3D8 scFv was post-treated, the survival rate of the 3D8 scFv untreated group (0 µM) in H1N1/PR8-infected cells was 63.2%, while the survival rate of the group post-treated with 5 µM of 3D8 scFv was 86.9% (H1N1/PR8(Post) in FIG. 7). Further, in H3N2/X-31-infected cells, the survival rate of the 3D8 scFv untreated group (0 µM) was about 62.7%, while the survival rate of the group post-treated with 5 µM of 3D8 scFv was 84.4% (H3N2/X-31(Post) in FIG. 7). In H1N1/H275Y-infected cells, the survival rate of the 3D8 scFv untreated group (0 µM) was about 58.4%, while the survival rate of all 3D8 scFv-treated groups was about 77.4% (H1N1/H275Y(Post) in FIG. 7) .

Treatment of 3D8 scFv before or after viral infection *in vitro* increased the viability of virus-infected cells by 20%.

### 7. Efficacy of antibody fragment to reduce viral RNA/protein expression

### (1) Measurement of the expression of viral RNA using qRT-PCR

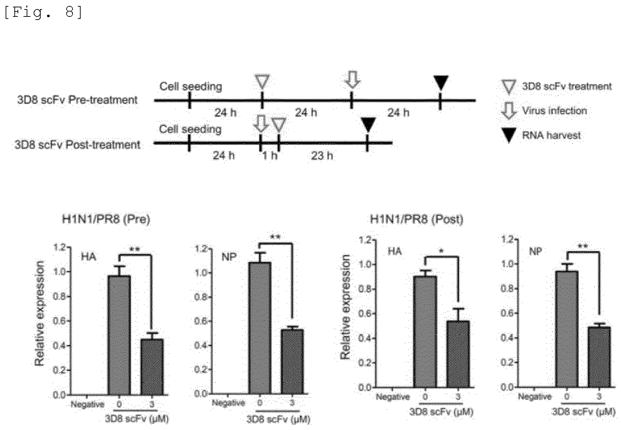

MDCK cells (4 × 10⁵) were cultured in 6-well plates and treated as follows: (i) 3D8 scFv pre-treatment: MDCK cells were treated with 3D8 scFv (3 µM) at 37° C under 5% CO₂ for 24 hours. Then, 3D8 scFv-treated cells were independently infected with three influenza virus strains (H1N1/PR8, H3N2/X-31 and H1N1/H275Y) (MOI = 0.1) in MEM-free medium at 37°C for 1 hour. Thereafter, the infection medium was removed and the cells were cultured in MEM-free medium (1% BSA) containing tosyl phenylalanyl chloromethyl ketone (TPCK)-treated trypsin (1 pg/ml) at 37° C under 5% CO₂ for 24 hours. (ii) 3D8 scFv post-treatment: MDCK cells were independently infected with three influenza virus strains (H1N1/PR8, H3N2/X-31 and H1N1/H275Y) (MOI = 0.1) in MEM-free medium at 37 °C for 1 hour, and then, the infection medium was removed. After replacing the medium with MEM-free medium (1% BSA) containing 3D8 scFv (3, 5, 7 µM) and TPCK-treated trypsin (1 pg/ml), the culturing was performed at 37°C under 5% CO₂ for 24 hours (the upper part of FIG. 8). Total RNA was isolated using TRI reagent (MRC, Montgomery, OH, USA) according to the manufacturer's instructions. RNA concentration was determined using a spectrophotometer. cDNA was synthesized using CellScript All-in-One 5X First Strand cDNA Synthesis Master Mix (Cell safe, Yongin, Korea) according to the manufacturer's protocol. Quantitative real-time PCR was performed using a SYBR Premix Ex Tag and Rotor-Gene Q system. Data were analyzed using Rotor-Gene Q series software version 2.3.1 (Qiagen, Chadstone, Victoria, Australia). Genes (HA and NP) were amplified using the primers shown in Table 3 below. GAPDH was amplified as an internal control.

**[TABLE 3]**

| **Gene** | **Forward(5'->3')** | **Reverse(5'->3')** | **Accession No.** |
|---|---|---|---|
| GAPDH (MDCK Cell Line) | AAC ATC ATC CCT GCT TCC ACT (SEQ ID NO: 17) | GGC AGG TCA GAT CCA CAA C (SEQ ID NO: 18) | NM_0010031 42.2 |
| GAPDH (Mouse) | GGC ATT GCT CTC AAT GAC AA (SEQ ID NO: 19) | TGT GAG GGA GAT GCT CAG TG (SEQ ID NO: 20) | NC_000072. 7 |
| Hemagglutin in (A/PuertoRi co/8/1934 (H1N1)) | AGT GCC CAA AAT ACG TCAG G (SEQ ID NO: 21) | CAG TCC ATC CCC CTT CAA TA (SEQ ID NO: 22) | NC_002017. 1 |
| Hemagglutin in (A/X-31 (H3N2)) | GAA AAT GGT TGG GAG GGA ATG (SEQ ID NO: 23) | GAT GGC TGC TTG AGT GCT TTT (SEQ ID NO: 24) | DQ874876.1 |
| Hemagglutin in (A/Californ ia/07/2009( H1N1)) | GGA ACG TGT TAC CCA GGA G (SEQ ID NO: 25) | GCT GCC GTT ACA CCT TTG TT (SEQ ID NO: 26) | NC_026433. 1 |
| Nucleoprote in (A/PuertoRi co/8/1934 (H1N1)) | ACC AAT CAA CAG AGG GCA TCT (SEQ ID NO: 27) | TGA TTT CGG TCC TCA TGT CAG (SEQ ID NO: 28) | NC_002019. 1 |
| Nucleoprote in (A/X-31 (H3N2)) | AAA ATC ATG GCG TCT CAA GGC (SEQ ID NO: 29) | CGG TGC ACA TTT GGA TGT AGA (SEQ ID NO: 30) | AB036779.1 |
| Nucleoprote in (A/Californ ia/07/2009( H1N1)) | CCA GAT CAG TGT GCA GCC TA (SEQ ID NO: 31) | GGC TTT GCA CTT TCC ATC ATT C (SEQ ID NO: 32) | NC_026436. 1 |
| Interferon beta (Mouse) | TTA CAC TCG CTT TGC CAT CCA A (SEQ ID NO: 33) | TCC CAC GTC AAT CTT TCC TCT T (SEQ ID NO: 34) | NC_000070. 6 |

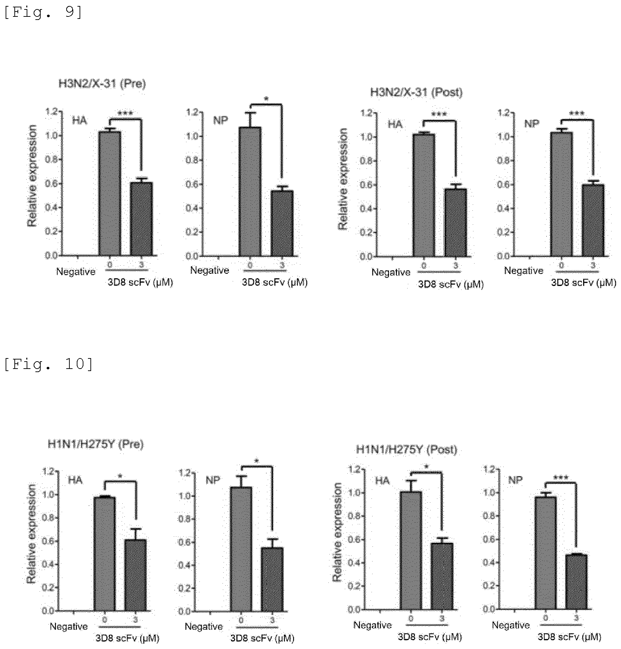

As a result of determining the expression of viral RNAs (HA and NP) at 24 hours after infection with three types of influenza virus, in the cells infected with H1N1/PR8, the viral HA and NP RNA expression of the 3D8 scFv pre-treated group was reduced by 50% or more compared to the 3D8 scFv untreated group (H1N1/PR8(Pre) in FIG. 8), and the viral HA and NP RNA expression of the 3D8 scFv post-treated group was reduced by 40% or more compared to the 3D8 scFv untreated group (H1N1/PR8 (Post) in FIG. 8). In the cells infected with H3N2/X-31, the viral HA and NP RNA expression of the 3D8 scFv pre-treated group was reduced by 40% or more compared to the 3D8 scFv untreated group (H3N2/X-31(Pre) in FIG. 9), and the viral HA and NP RNA expression of the 3D8 scFv post-treated group was also reduced by 40% or more compared to the 3D8 scFv untreated group (H3N2/X-31(Post) in FIG. 9). Further, in the cells infected with H1N1/H275Y, the viral HA and NP RNA expression of the 3D8 scFv pre-treated group was reduced by 35% or more compared to the 3D8 scFv untreated group (H1N1/H275Y(Pre) in FIG. 10), and the viral HA and NP RNA expression of the 3D8 scFv post-treated group was also decreased by 40% or more compared to the 3D8 scFv untreated group (H1N1/H275Y(Post) in FIG. 10).

### (2) Measurement of viral protein expression using immunocytochemistry (ICC)

MDCK cells (2 × 10⁴) were cultured in 8-well chamber slides. 3D8 scFv treatment and virus infection (H1N1/PR8 and H1N1/H275Y) were performed in the same manner as described in (1) above (the upper part of FIG. 8). The slides were washed twice with PBS and fixed for 15 minutes using methanol cold at 25°C. The sample was permeabilized with Intracellular Staining Perm wash buffer for 5 minutes. After blocking with PBST containing 1% BSA for 1 hour, the cells were incubated with monoclonal mouse anti-3D8 scFv antibody or polyclonal rabbit anti-HA antibody (1:1000 dilution, Invitrogen) at 4 °C for 24 hours. The cells were then cultured with goat anti-mouse IgG Alexa Fluor 488 secondary antibody or goat anti-rabbit IgG TRITC secondary antibody (1:1000 dilution, Abcam), respectively, at 25° C for 1 hour. The slides were mounted using anti-fade mounting medium containing DAPI and the cells were visualized using a Zeiss LSM 700 confocal microscope.

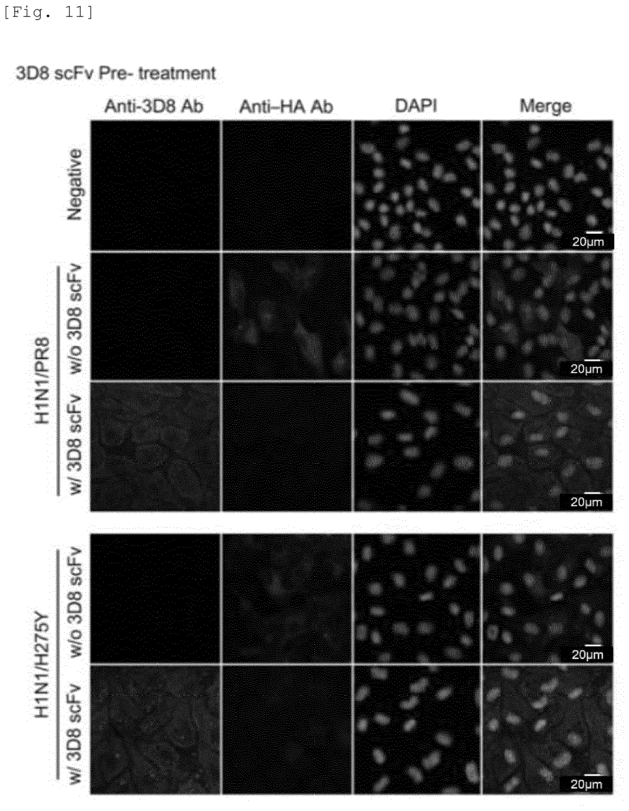

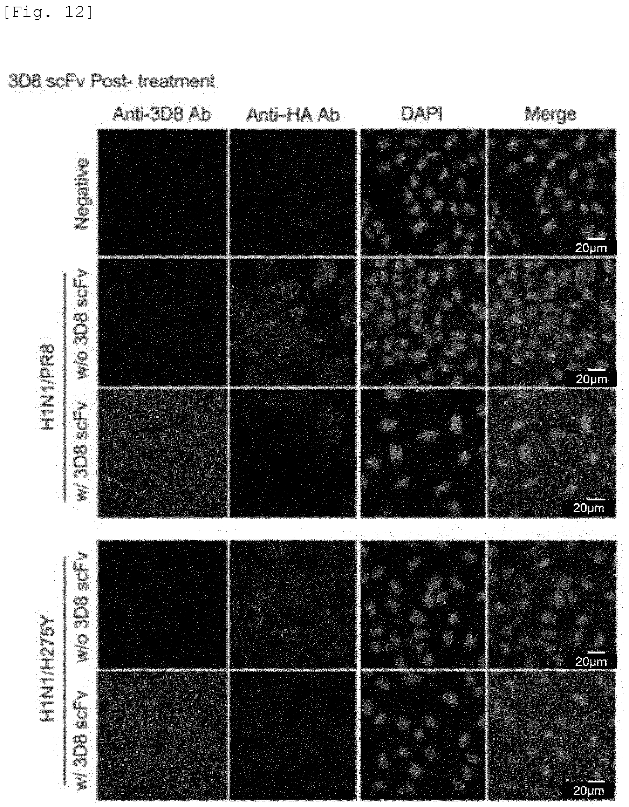

Immunofluorescence analysis was performed to confirm the presence of viral HA protein and 3D8 scFv. In the 3D8 scFv untreated group of H1N1/PR8-infected cells, viral HA protein was detected in the cytoplasm (red signal), whereas in the 3D8 scFv pre-treated group, no viral HA protein was detected (H1N1/PR8 in FIG. 11). 3D8 scFv (green signal) was found in the cytoplasm of the 3D8 scFv-treated group. The 3D8 scFv pre-treated group of H1N1/H275Y-infected cells had a lower detection signal of viral HA protein than the 3D8 scFv untreated group (H1N1/H275Y in FIG. 11). Further, in the H1N1/PR8 and H1N1/H275Y-infected cells, the 3D8 scFv post-treated group had a lower signal of viral HA protein than the 3D8 scFv untreated group (FIG. 12).

### 8. Antibody fragment in-vivo antiviral activity test [150] (1) Clinical antiviral efficacy in H1N1/H275Y infected mice

In order to assess clinical antiviral effects of 3D8 scFv, an experiment was performed on mice as an experimental in *vivo* model. Six-week-old male specific pathogen-free (SPF) BALB/c mice (DBL; body weight 18 to 20 g) were housed under standard laboratory conditions. These mice were divided into 5 groups of 8 to 10 mice.

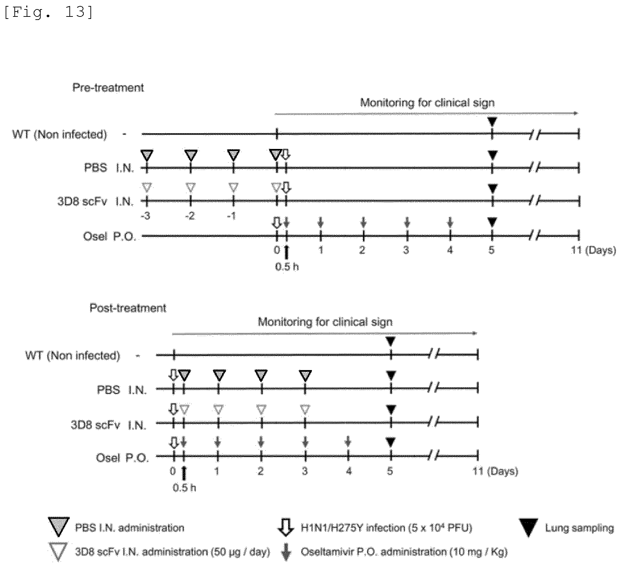

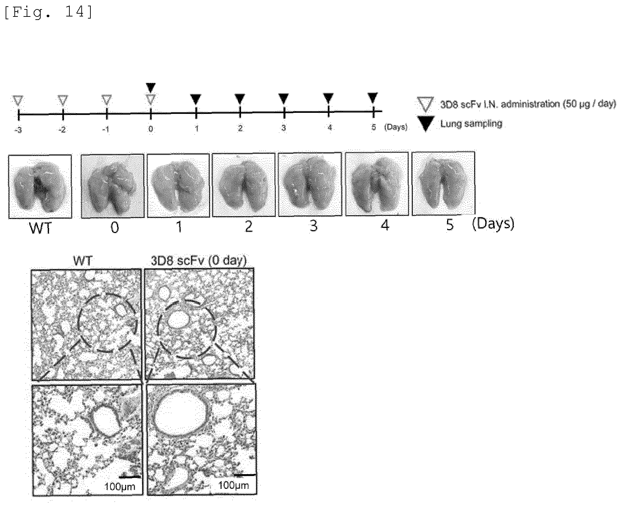

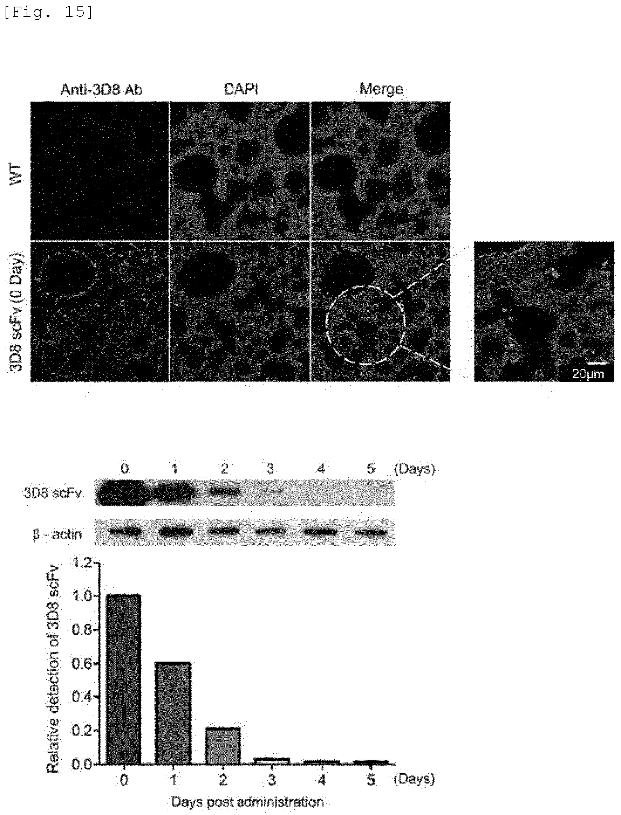

PBS treatment prior to virus infection in each group of mice; PBS treatment after viral infection; 3D8 scFv treatment prior to viral infection; 3D8 scFv treatment after viral infection; and Oseltamivir phosphate (Sigma-Aldrich, ST. Louis, MO, USA) was orally administered for 5 days after viral infection. Mice in each group were intra-nasally infected with 50 µl lethal dose of 50% H1N1/H275Y virus (5 × 10⁴ PFU). Mice were infected with H1N1/H275Y and monitored daily for clinical signs (weight change, cough, slouched back, coarse hair, flocking (fever), abnormal secretions, survival, etc.) until day 11 (FIG. 13). For reference, after administration of 3D8 scFv, mice did not show abnormal clinical signs and, in the aspect of histopathology, abnormal changes in the lung parenchyma and its stroma did not appear (FIG. 14). Further, 3D8 scFv was found to be localized to the epithelial lining of bronchioles and alveoli for 48 hours after administration (FIG. 15).

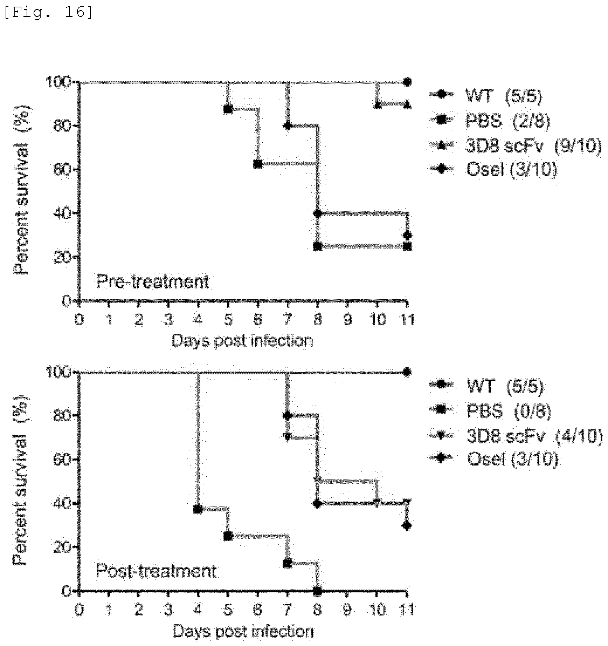

Only 25% of the PBS pre-treated group survived, whereas 90% of the 3D8 scFv pre-treated group survived. 40% of the 3D8 scFv post-treated group survived but none of the PBS post-treated groups survived. 30% of the oseltamivir-treated group survived (FIG. 16).

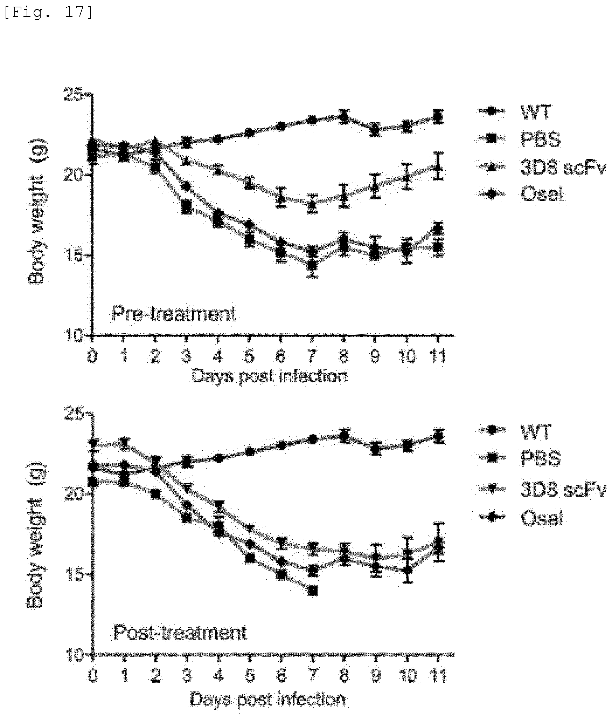

For body weight change, the average body weight of the PBS pre-treated group was 21.1 g after infection 0 day (0 dpi), and decreased by 27% to 15.5 g after infection 11 day (11 dpi). The average body weight of the 3D8 scFv pre-treated group was 22.2 g at 0 dpi, and decreased by about 18% to 18.2 g at 7 dpi. However, the average weight rebounded to 20.6 g at 11 dpi. In the oseltamivir-treated group, the average body weight was 21.8 g at 0 dpi and decreased by 30% at 7 dpi, but rebounded at 11 dpi. In the PBS post-treated group, the average body weight was 20.8 g at 0 dpi, but no mice survived at 8 dpi. The average body weight of the 3D8 scFv post-treated group was 23 g at 0 dpi and decreased by about 30% to 16 g at 10 dpi, but the average body weight was recovered thereafter (FIG. 17).

From this, it can be confirmed that the treatment of 3D8 scFv increases the viability of virus-infected cells and rapidly recovers the initial weight loss caused by the virus attack.

### (2) Morphological analysis of lungs of influenza A virus-infected mice

Lung tissues from each group of mice (5 dpi) in (1) above were collected and morphological analysis of lung lesion scores was performed by visually observing.

Each lung lobe was assigned with a number reflecting the approximate percentage of a total lung volume. The total score for all lobes formed an estimate of total percentage of the lungs with visible pneumonia. For morphological analysis of microscopic lung lesion scores, lung tissue was fixed in 10% neutral buffered formalin. After fixation, the tissue was dehydrated through a series of alcohol solutions and xylene, and embedded in paraffin wax. Sections (5 um) were prepared from each tissue, and hematoxylin and eosin (HE) staining was performed. The prepared tissue slides were examined in a blinded fashion and scored for the estimated severity of interstitial pneumonia as follows: 0, no lesion; 1, mild interstitial pneumonia; 2, moderate multifocal interstitial pneumonia; 3, moderate diffuse interstitial pneumonia; and 4, severe interstitial pneumonia. For morphological analysis of IHC (Immunohistochemistry), sections were analyzed with NIH Image J 1.43m Program (https://imagej.nih.gov/ij) to obtain quantitative data. Ten fields were randomly selected and the number of positive cells per unit area (0.25 mm²) was determined, followed by calculating an average value thereof.

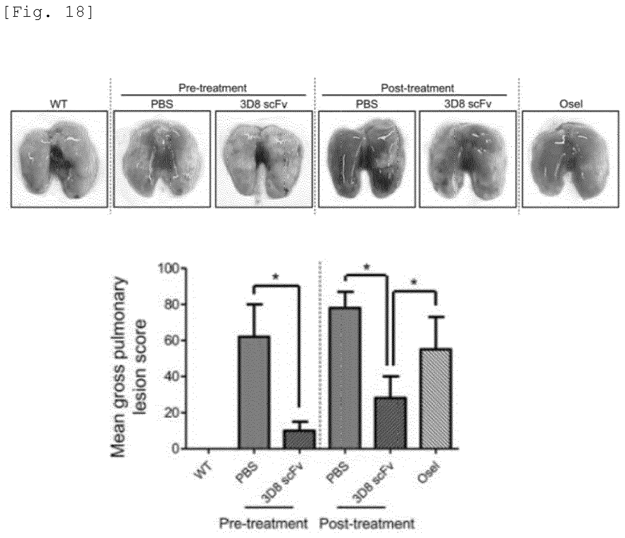

Visible lesions were mainly present in the mesenchymal, caudate and accessory lobes. Pathologically, the lungs of the 3D8 scFv pre-treated group were not significantly different from those of the non-virally infected WT group. On the other hand, significant lung congestion and tan enhancement were observed in the PBS pre-treated group and the oseltamivir-treated group (the upper part of FIG. 18). The 3D8 scFv pre- and post-treated groups had significantly lower lung lesion severity scores than the PBS and oseltamivir-treated groups (P < 0.05). Among the 3D8 scFv-treated groups, the total lung lesion score was significantly lower in the 3D8 scFv pre-treated group than in the 3D8 scFv post-treated group (P < 0.05). The oseltamivir-treated group had a significantly lower total lung lesion score than the PBS-treated group (P < 0.05) (the lower part of FIG. 18).

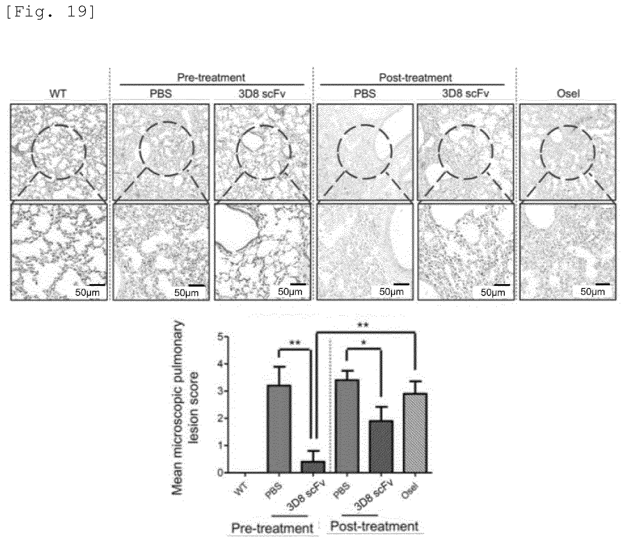

The microscopic lesion was characterized by a thickened alveolar septum with increased numbers of interstitial macrophages and lymphocytes in the PBS and oseltamivir treated groups. The lungs of 3D8 scFv pre-treated and WT groups were normal (the upper part of FIG. 19). The 3D8 scFv pre-treated group had significantly lower microscopic lesion scores than the other groups (P < 0.01), and the 3D8 scFv post-treated group had significantly lower microscopic lesion scores than the PBS and oseltamivir-treated groups (P < 0.05). There was no significant difference in the microscopic lung lesion score between the PBS-treated group and the oseltamivir-treated group (the lower part of FIG. 19).

### (3) Measurement of viral RNA and interferon levels in influenza A virus-infected mouse lung

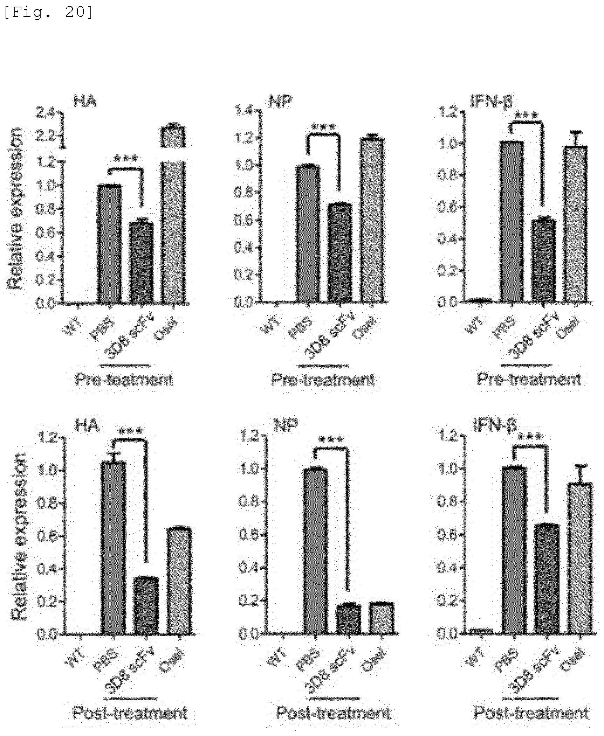

Viral RNA and interferon levels were measured in the lungs of mice. Total RNA was extracted from lysed mouse lung tissue (5 dpi) samples using TRI reagent according to the manufacturer's instructions. RNA concentration was determined using a spectrophotometer. cDNA was synthesized using CellScript All-in-One 5x First Strand cDNA Synthesis Master Mix according to the manufacturer's protocol. The genes (HA, NP and IFN-β) were amplified with the primers in Table 3 using qRT-PCR as previously described. GAPDH was amplified as an internal control. In the lungs of the 3D8 scFv pre-treated group, as evidenced by the reduced expression of HA (68%), NP (71%) and IFN-β (51%) in the lungs of the 3D8 scFv pre-treated group compared to the PBS pre-treated group, the expression of viral RNA and IFN-β was decreased compared to other groups. Expression of viral RNA in the lungs of the 3D8 scFv post-treated group was as low as 35% (HA) and 18% (NP) (FIG. 20).

### (4) Confirmation of virus titer in influenza A virus-infected mouse lung

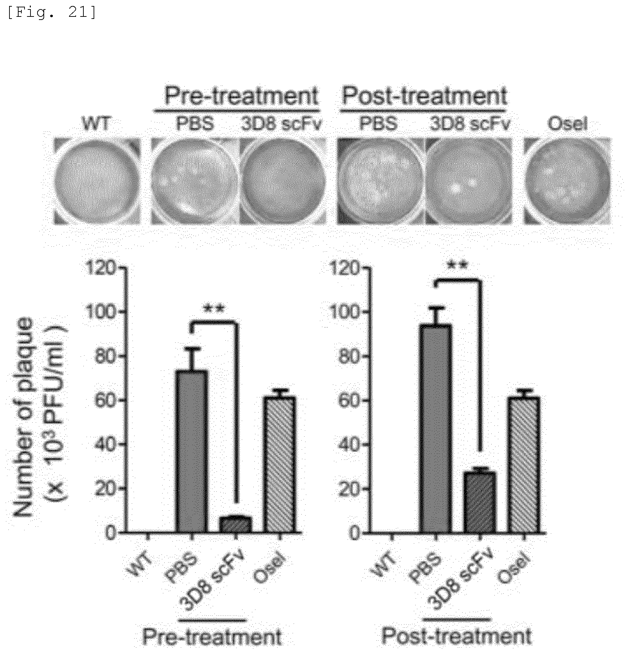

Further, virus titers were investigated in the lungs of influenza A virus-infected mice. Specifically, mouse lung tissues were homogenized in 1 ml of MEM-free medium using TissueRuptor (Qiagen, Chadstone, Victoria, Australia). Then, a homogenate was obtained by centrifugation at 6,000 × g and at 4°C for 5 minutes. Confluent MDCK cells were cultured in 12-well plates. After infecting the tissue supernatant (1:1000 dilution) in MEM-free medium at 37 °C for 1 hour, the infection medium was removed. The medium was replaced with 1× Dulbecco's modified eagle medium containing TPCK-treated trypsin (1 pg/ml) and 1% agarose. Cells were further incubated under 5% CO₂ at 37°C for 3 days. Cell monolayers were fixed with 4% formaldehyde and stained with 0.5% crystal violet for visualization. The viral titer of the lungs was reduced by about 70% in the 3D8 scFv pre-treated group compared to the PBS pre-treated group (FIG. 21). The viral titer of the lungs was reduced by about 60% in the 3D8 scFv post-treated group compared to the PBS post-treated group (FIG. 21).

### (5) Measurement of viral protein expression using immunohistochemistry (IHC)

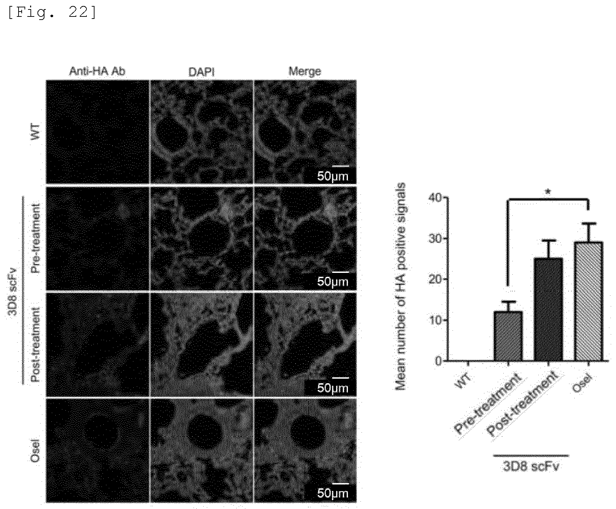

After de-paraffinizing and rehydrating the tissue sections, antigen recovery was performed at 95 °C for 20 minutes using citrate buffer. Slides were washed with TBST (TBS, 0.025% Triton X-100) and blocked with 10% fetal bovine serum + 1% BSA solution (in TBST) for 2 hours. Tissue sections were incubated with polyclonal rabbit anti-HA antibody (1:750 dilution) overnight at 4 °C. The tissue slides were then each incubated with goat anti-rabbit IgG TRITC secondary antibody (1:1000 dilution) at 25 °C for 1 hour. Anti-fade mounting medium containing DAPI was put on tissue section slides, a cover glass was put on, and cells were visualized using an LSM 700 Zeiss confocal microscope. A signal of viral HA was reduced in an alveolar epithelial cell inner layer (the left side in FIG. 22), and the number of distinct positive signals was significantly lower in the 3D8 scFv pre-treated group compared to the oseltamivir-treated group (P < 0.05) (the right side in FIG. 22).

### 9. Statistical analysis

Continuous data were analyzed by a one-way analysis of variance (ANOVA). When the ANOVA analysis was significant (P < 0.05), pairwise Tukey's adjustment was further performed. Discrete data were analyzed by Chisquare and Fisher's exact test. If necessary, the relationship between the data was assessed using Pearson's correlation coefficient and/or Spearman's rank correlation coefficient. Values of P < 0.05 were considered significant. All statistical analyzes were performed using GraphPad Prism software (GraphPad Software, San Diego, CA, USA).

From the above results, it was confirmed that 3D8 scFv not only exhibits prophylactic effects of protecting the host from viral infection, but also exhibits therapeutic effects by alleviating excessive inflammation and potential additional etiology after viral infection.

The description of the present invention is for illustrative purposes, and those of ordinary skill in the art to which the present invention pertains will understand that it can be easily modified into other specific forms without changing the technical spirit or essential features of the present invention. Accordingly, it should be understood that the embodiments described above are illustrative in all respects and not restrictive.

The scope of the present invention is indicated by the following appended claims, and all changes or modifications derived from the meanings and scope of the claims and their equivalents should be construed as being included in the scope of the present invention.

## Claims

1. An antiviral composition for influenza A virus, comprising an antibody or fragment thereof, which includes:
a heavy chain CDR1 (VH CDR1) represented by SEQ ID NO: 1; VH CDR2 represented by SEQ ID NO: 2; VH CDR3 represented by SEQ ID NO: 3; a light chain CDR1 (VL CDR1) represented by SEQ ID NO: 4; VL CDR2 represented by SEQ ID NO: 5; and VL CDR3 represented by SEQ ID NO: 6.

2. The antiviral composition according to claim 1, wherein the antibody or fragment thereof includes a heavy chain variable region comprising an amino acid sequence of SEQ ID NO: 7.

3. The antiviral composition according to claim 1, wherein the antibody or fragment thereof includes a light chain variable region comprising an amino acid sequence of SEQ ID NO: 8.

4. The antiviral composition according to claim 1, wherein the fragment is scFv comprising an amino acid sequence of SEQ ID NO: 9.

5. The antiviral composition according to claim 1, wherein the influenza A virus is at least one selected from the group consisting of influenza A virus H1 subtype, influenza A virus H2 subtype, influenza A virus H5 subtype and influenza A virus H6 subtype, influenza A virus H3 subtype, influenza A virus H4 subtype, influenza A virus H14 subtype, and variants thereof.

6. The antiviral composition according to claim 1, wherein the influenza A virus is at least one selected from the group consisting of influenza A virus N1 subtype, influenza A virus N2 subtype, and variants thereof.

7. The antiviral composition according to claim 1, wherein the influenza A virus is at least one of H1N1 subtype, H3N2 subtype, and variants thereof.

8. The antiviral composition according to claim 1, wherein the influenza A virus is an oseltamivir-resistant influenza A virus.

9. A pharmaceutical composition for prevention or treatment of influenza A virus-infectious diseases, comprising the composition of any one of claims 1 to 8.

10. The pharmaceutical composition according to claim 9, which is used for treating influenza A virus-infectious disease.

11. A food composition for prevention or treatment of influenza A virus-infectious diseases, comprising the composition of any one of claims 1 to 8.

12. The food composition according to claim 11, wherein the composition is used for improving influenza A virus-infectious diseases.
